# EUROPEAN PATENT APPLICATION

(11) **EP 4 744 599 A1**
(43) Date of publication of application: **20.05.2026**
(21) Application number: 24936192.4
(22) Date of filing: 26.12.2024
(51) Int. Cl.: A61B 17/064

(54) **COMPRESSION FOLDABLE FULL-SUTURE INTERFACE SCREW**

(30) Priority: 23.09.2024 CN 202411320673
(71) Applicant: STAR SPORTS MEDICINE CO., LTD., Beijing 100176 (CN)
(72) Inventor: DONG, Wenxing, Daxing District, Beijing 100176 (CN); YIN, Jichen, Daxing District, Beijing 100176 (CN); YAN, Kepan, Daxing District, Beijing 100176 (CN); LI, Zhengrong, Daxing District, Beijing 100176 (CN); GUO, Tingting, Daxing District, Beijing 100176 (CN)
(74) Representative: Angerhausen, Christoph
(86) International application number: PCT/CN2024/142928
(87) International publication number: WO 2026/060862

(57) **Abstract**

The present application discloses a compressible and foldable all-suture interface screw, and the compressible and foldable all-suture interface screw includes a deformable fixation body, a pull bolt, and a top bolt; the deformable fixation body includes a flexible pipe and several rigid blocks fixed to an outer wall of the flexible pipe, the flexible pipe is made of a textile fabric, the rigid block is an isosceles trapezoidal column, an end surface of the rigid block close to the flexible pipe is a connection surface, adjacent two of the rigid blocks form a folding unit, long side surfaces of two of the rigid blocks in a same one of the folding units are opposite to each other, and under a condition that force is applied to the folding unit along the length direction of the flexible pipe, the folding unit drives the flexible pipe to deform and move to cause two of the connection surfaces to be close to each other; the bolt rod of the pull bolt passes through the flexible pipe and the top bolt in sequence, the top bolt is slidable relative to the bolt rod of the pull bolt, and the deformable fixation body is sandwiched between a bolt head and a top bolt.

## Description

### CROSS-REFERENCE TO RELATED APPLICATION

The present application claims priority to Chinese Patent Application CN202411320673.5 filed on 23 September, 2024, entitled "COMPRESSIBLE AND FOLDABLE ALL-SUTURE INTERFACE SCREW", which is incorporated herein by reference in its entirety.

### TECHNICAL FIELD

The present application relates to the technical field of medical devices, and particularly, to a compressible and foldable all-suture interface screw.

### BACKGROUND

In the related art, an interface screw is usually used to reattach and fix the damaged part and the bone, and a "bone-graft interface" and a "graft-screw interface" are formed between the bone and the graft and the graft and the screw. The graft located in the middle of two interfaces mainly relies on the screw teeth to compress the bone tunnel to achieve the fixation purpose. However, in terms of structure, due to the small fixed contact surface between the interface screw and the bone and the bone yielding, the interface screw will loosen, leading to the failure of the overall repair and fixation system; also, ordinary mechanical parts cannot be used in the human body to avoid the connection instability caused by the vibration and the alternating load generated by daily activities of the human body. In terms of material, traditional interface screws are usually made of metal materials which can't be absorbed by the human body, and the great stiffness difference in the metal and the bone easily leads to the unnecessary damage to the bone. Although non-metal materials such as inorganic ceramics, polymer materials, and magnesium-based zinc alloys have appeared nowadays to reduce the problem of tissue growth, the toughness of the materials is insufficient, and the materials easily fracture when "screwed"; and even after the materials are screwed in, long-term stress concentration will cause fracture and failure.

In conclusion, medical grafts need to satisfy requirements for mechanical strength, fixation stability, and biocompatibility at the same time. In addition, iatrogenic injuries, such as relatively large bone damage caused by drilling due to relatively large screw diameter, temporary damage to irrelevant tissue caused by screwing the screw during surgery, and poor tissue healing caused by non-absorbent materials, need to be avoided.

At present, the interface screw used to fix the grafts in medical devices is mostly a structurally fixed interface screw or an anchor product. These products have a monotonous structural shape and poor adaptability to different human body bone tunnels, and are implanted mostly by screwing or knocking. Therefore, the requirements for the structure of the fixture and the surgical operation are very high. Although the fixture products with all suture have appeared at present and solved a part of the technical problems such as small bone damage, in practice, due to the limitation of the environment of the bone tunnel, the interface screw will not be completely deformed into an ideal state, and the deformation and distortion of the interface screw will reduce the strength of the fixture, leading to the unstable fixation.

### SUMMARY

One of the purposes of the present application is to provide a compressible and foldable all-suture interface screw for providing the folding unit which is fixed and stable and the compressible and foldable structure which is bi-directionally stable, so as to ensure long-term stable operation of the deformable fixation body.

To achieve this purpose, the following technical solution is used in embodiments of a first aspect of the present application:

a compressible and foldable all-suture interface screw including a deformable fixation body, a pull bolt, and a top bolt, where the deformable fixation body includes a flexible pipe and a plurality of rigid blocks fixed to an outer wall of the flexible pipe, the flexible pipe is made of a textile fabric, the rigid block is an isosceles trapezoidal column in shape, a thickness direction of the rigid block is same as a radial direction of the flexible pipe, a length direction of the flexible pipe is perpendicular to a long side surface of the rigid pipe, an end surface of the rigid block close to the flexible pipe is a connection surface, adjacent two of the rigid blocks form a folding unit, long side surfaces of two of the rigid blocks in a same one of the folding units are opposite to each other, and under a condition that force is applied to the folding unit along the length direction of the flexible pipe, and the folding unit drives the flexible pipe to deform and move to cause two of the connection surfaces to be close to each other; the pull bolt has a bolt head and a bolt rod fixedly connected to one end of the bolt head, the bolt rod passes through the flexible pipe and the top bolt in sequence, the top bolt is slidable relative to the bolt rod, a length direction of the bolt rod is same as a length direction of the deformable fixation body, and the deformable fixation body is sandwiched between the bolt head and the top bolt.

According to the embodiments of the first aspect of the present application, the rigid blocks are distributed over the outer wall of the flexible pipe, and a cross section of the flexible pipe is a hexagon in shape.

According to any one of the previous embodiments of the first aspect of the present application, the flexible pipe consists of a partition part and a carrying part, the partition part is for partitioning all of the carrying parts one by one, each of the carrying parts is provided with one of the rigid blocks, a shape of each of the carrying parts is same as a shape of a projection of a cross section of the rigid block on the carrying part, and a dimension of each of the carrying parts is provided to scale.

According to any one of the previous embodiments of the first aspect of the present application, the partition part and the carrying part are integrally woven, one of the partition part and the carrying part consists of a knit stitch coil, and the other of the partition part and the carrying part consists of a purl stitch coil.

According to any one of the previous embodiments of the first aspect of the present application, the outer contour of the partition part enclosing one of the folding units is a regular hexagon in shape.

According to any one of the previous embodiments of the first aspect of the present application, the rigid block includes a filler and a capsule, the capsule is fixedly connected to the outer wall of the flexible pipe, the capsule and the flexible pipe enclose a filling cavity, and the filling cavity is filled with the filler.

According to any one of the previous embodiments of the first aspect of the present application, the capsule is made of a textile fabric, the rigid block further includes a overlock stitch for sewing the capsule to the outer wall of the flexible pipe, and the overlock stitch forms equally spaced stitching points along an edge of the capsule.

According to any one of the previous embodiments of the first aspect of the present application, the filler is fibrous, and a volume of the filler is between 30% and 80% of a maximum volume of the filling cavity. As an optional technical solution of the compressible and foldable all-suture interface screw, the top bolt is provided with an avoidance hole through the top bolt, and a hole wall of the avoidance hole is slidably fitted to an outer wall of the bolt rod.

According to any one of the previous embodiments of the first aspect of the present application, the top bolt is a hollow tubular component, the top bolt includes a bolt pipe and a bolt ring fixedly connected to one end of the bolt pipe, a cross section of the bolt ring covers a cross section of the bolt pipe, and the deformable fixation body is sandwiched between the bolt head and the bolt ring.

The following are beneficial effects of the present application.

The deformable fixation body of the compressible and foldable all-suture interface screw consists of the flexible pipe and the rigid blocks, and can be deformed and moved under a condition that force is applied along the length direction of the deformable fixation body, so that two connection surfaces in the folding unit are close to each other to achieve the accurate control over the deformation of the deformable fixation body. The rigid blocks are grouped and limited by the folding unit, so that the long side surfaces of two rigid blocks are provided oppositely on the hexagonal folding unit, and each folding unit is evenly distributed over the flexible pipe. The pull bolt and the top bolt serve the function of assisting the folding and deformation of the deformable fixation body, and by fitting to the pull bolt and the top bolt, the deformable fixation body can be sandwiched between the pull bolt and the top bolt, which can provide desired stability and reliability for the overall structure of the compressible and foldable all-suture interface screw. The deformable fixation body is fitted to the shaft holes of the pull bolt and the top bolt, so as to assist folding and deforming of the deformable fixation body based on the relative axial movement between the pull bolt and the top bolt. After the deformation of the deformable fixation body is completed, the pull bolt and the top bolt are withdrawn to fix the deformable fixation body to the bone tunnel, and the pull bolt and the top bolt are withdrawn after the auxiliary operation is completed. The compressible and foldable all-suture interface screw is folded and deformed by the deformable fixation body, so that the diameter of the deformable fixation body after deformation is increased along the radial direction, and the deformable fixation body has a laminated structure along the axial direction. Further, the flexible pipe is made of the textile fabric, so that the deformable fixation body may be in contact with the bone tunnel through the flexible object, thereby increasing the roughness of the surface of the deformable fixation body, increasing the friction between the compressible and foldable all-suture interface screw and the bone tunnel, and improving the use effect. Therefore, the movement of the deformable fixation body may be restricted by the friction between the bone surface and the deformable fixation body, so that the deformable fixation body is not easily expanded after compression and folding, thereby ensuring the long-term stable operation of the deformable fixation body. With the design that the deformable fixation body after compression and folding is in contact with the bone tunnel, the contact area between the deformable fixation body and the bone tunnel is increased, the compressing force between the deformable fixation body and the surface bone is increased, and the toughness of the deformable fixation body 300 itself is increased, so that the damage to the bone in practice is reduced, the self-adaptability to the bone tunnel is increased, and the fixation strength and the fixation stability are increased, thereby reducing the risk of surgical failure, helping to simplify the practical clinical surgical operation, and providing stable post-surgical repair effect. The compressible and foldable all-suture interface screw is provided with the folding unit which is fixed and stable and the compressible and foldable structure which is bi-directionally stable, ensuring the long-term stable operation of the deformable fixation body with the preset deformation solution for the deformable fixation body.

### BRIEF DESCRIPTION OF THE DRAWINGS

Fig. 1 is a schematic structural view of a compressible and foldable all-suture interface screw according to embodiments of the present application;
Fig. 2 is a front view of a compressible and foldable all-suture interface screw according to embodiments of the present application;
Fig. 3 is a sectional view of a compressible and foldable all-suture interface screw according to embodiments of the present application;
Fig. 4 is a schematic structural view of a deformable fixation body according to embodiments of the present application;
Fig. 5 is a front view of a deformable fixation body according to embodiments of the present application;
Fig. 6 is a cross-sectional view of a deformable fixation body according to embodiments of the present application;
Fig. 7 is a sectional view of a deformable fixation body according to embodiments of the present application;
Fig. 8 is a partial enlarged view of A in Fig. 7;
Fig. 9 is a schematic structural view of a deformable fixation body after deformation according to embodiments of the present application;
Fig. 10 is a sectional view of a deformable fixation body after deformation according to embodiments of the present application;
Fig. 11 is a schematic structural view of a rigid block according to embodiments of the present application;
Fig. 12 is a sectional view of a rigid block and a part of flexible pipes according to embodiments of the present application;
Fig. 13 is a schematic structural view of a top bolt according to embodiments of the present application;
Fig. 14 is a sectional view of a top bolt according to embodiments of the present application;
Fig. 15 is a schematic structural view of a pull bolt according to embodiments of the present application;
Fig. 16 is a sectional view of a pull bolt according to embodiments of the present application;
Fig. 17 is a schematic structural view of a pull bolt and a top bolt according to embodiments of the present application;
Fig. 18 is a sectional view of a pull bolt and a top bolt according to embodiments of the present application;
Fig. 19 is a front view of a folding unit according to embodiments of the present application;
Fig. 20 is a sectional view of a folding unit according to embodiments of the present application;
Fig. 21 is a sectional view 1 of a folding unit during a folding process according to embodiments of the present application;
Fig. 22 is a sectional view 2 of a folding unit during a folding process according to embodiments of the present application;
Fig. 23 is a sectional view of a folding unit after folding according to embodiments of the present application;
Fig. 24 is a partial unfolded view of a deformable fixation body according to embodiments of the present application;
Fig. 25 is a sectional view 1 of a compressible and foldable all-suture interface screw implantation method according to embodiments of the present application;
Fig. 26 is a sectional view 2 of a compressible and foldable all-suture interface screw implantation method according to embodiments of the present application;
Fig. 27 is a sectional view 3 of a compressible and foldable all-suture interface screw implantation method according to embodiments of the present application;
Fig. 28 is a sectional view 4 of a compressible and foldable all-suture interface screw implantation method according to embodiments of the present application; and
Fig. 29 is a sectional view 5 of a compressible and foldable all-suture interface screw implantation method according to embodiments of the present application.

Reference signs:
100: Pull bolt; 110: Bolt rod; 120: Bolt head; 121: Frustum;
200: Top bolt; 210 Bolt pipe; 220: Bolt ring; and
300: Deformable fixation body; 310: Flexible pipe; 311: Carrying part; 312: Partition part; 320: Rigid block; 321: Filler; 322: Capsule; 323: Overlock stitch.

### DETAILED DESCRIPTION

Technical solutions of the present application will be described clearly and completely with reference to drawings below. Obviously, the described embodiments are a part, but not all, of the embodiments of the present application. Based on the embodiments of the present application, all other embodiments obtained by those ordinary skilled in the art without any creative work shall fall within the protection scope of the present application.

In the description of the present application, it should be noted that the orientation or positional relationship indicated by the terms "center", "upper", "lower", "left", "right", "vertical", "horizontal", "inner", and "outer" is based on the orientation or positional relationship shown in the drawings, and is only for facilitating the description of the present application and simplifying the description, but not for indicating or implying that the referred apparatus or element must have a particular orientation, or be configured and operated in a particular orientation, which cannot be understood as the limitation to the present application. In addition, the terms "first" and "second" are for descriptive purposes only, and cannot be understood as indicating or implying relative importance. The terms "first position" and "second position" are two different positions, and the first feature is "over", "above" and "on" the second feature indicates that the first feature is directly above or diagonally above the second feature, or only indicates that the first feature is horizontally higher than the second feature. The first feature is "below", "under" and "beneath" the second feature indicates the first feature is directly under or obliquely under the second feature, or only indicates that the first feature is horizontally lower than the second feature.

In the description of the present application, it should be noted that, unless otherwise explicitly specified and limited, the terms "installed", "connected", "connection" should be understood in a broad sense, for example, they may refer to a fixed connection, a detachable connection, or an integral connection, or may be a mechanical connection or an electrical connection, or may be a direct connection, an indirect connection via an intermediate medium, or an internal communication between two elements. For those of ordinary skill in the art, the specific meanings of the above terms in the present application may be understood in accordance with specific conditions.

The embodiments of the present application are described in detail below, and the examples of the embodiments are shown in the drawings. In the drawings, the same or similar reference numerals represent the same or similar elements or elements having the same or similar functions throughout. The embodiments described below with reference to the drawings are exemplary and are merely for explaining the present application, and should not be understood as the limitation to the present application.

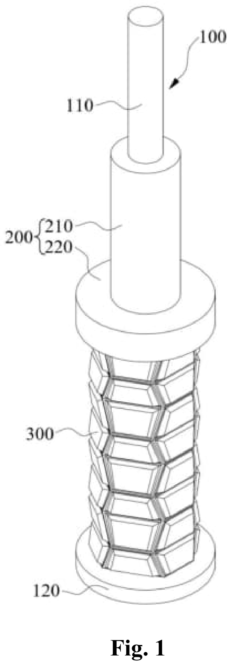

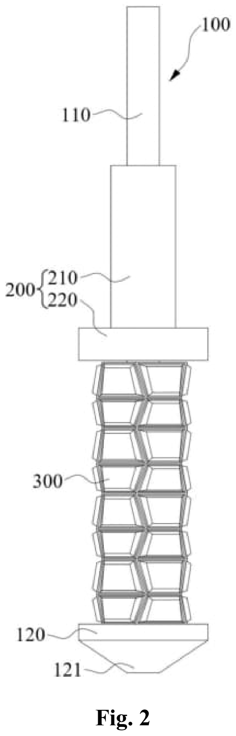

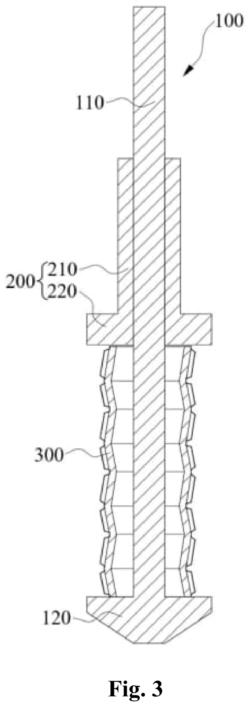

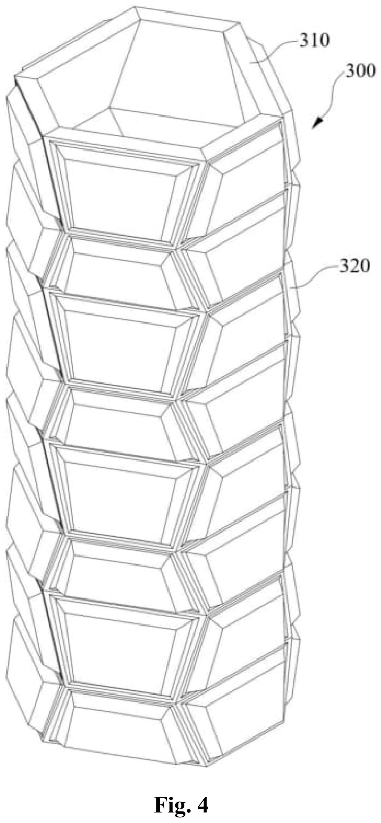

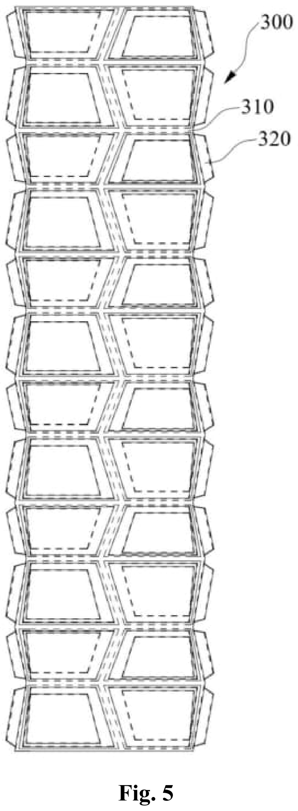

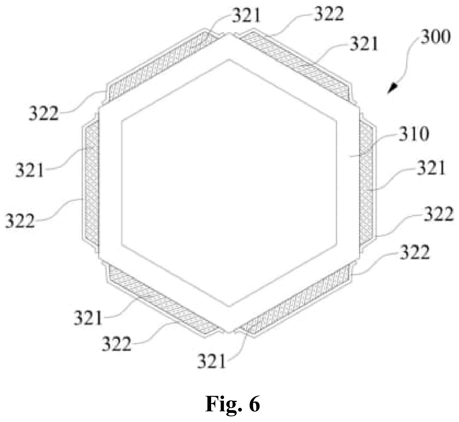

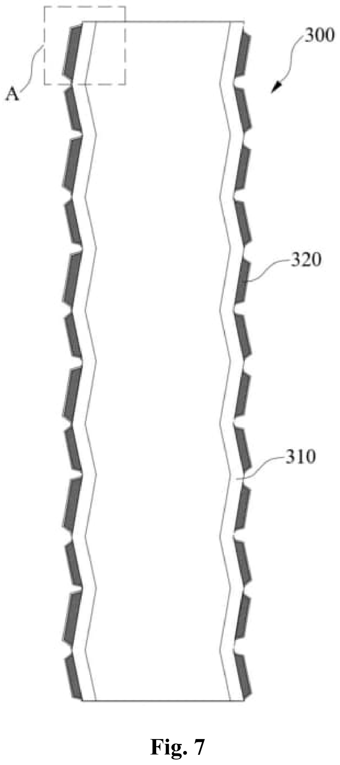

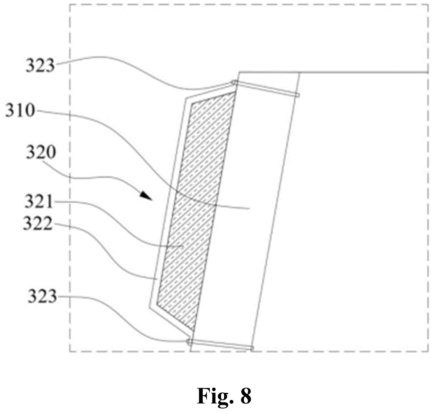

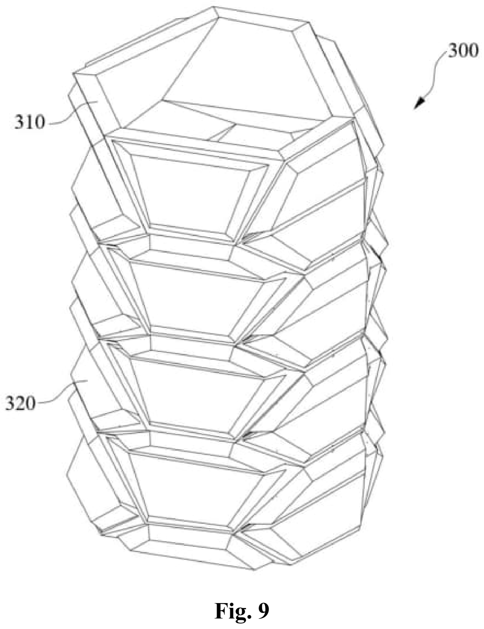

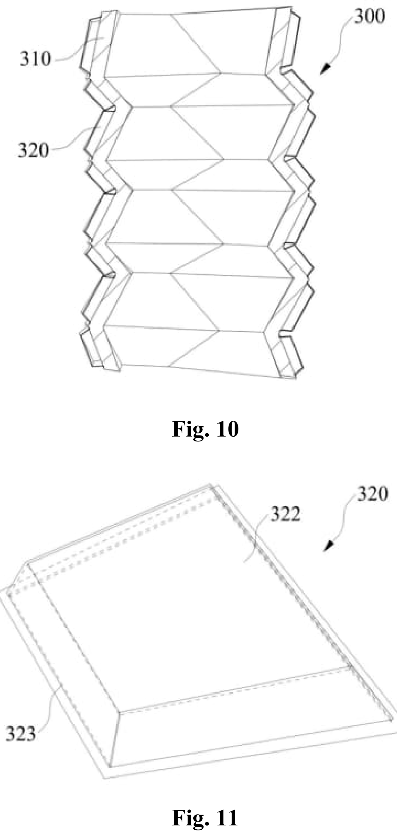

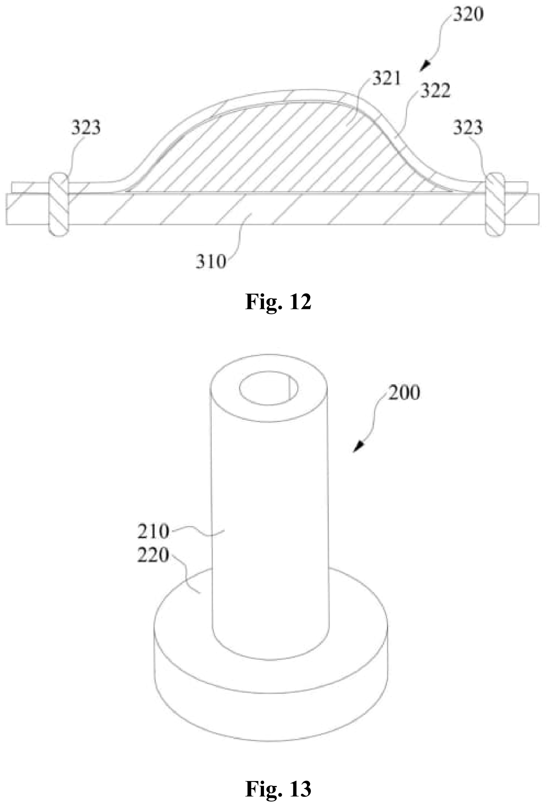

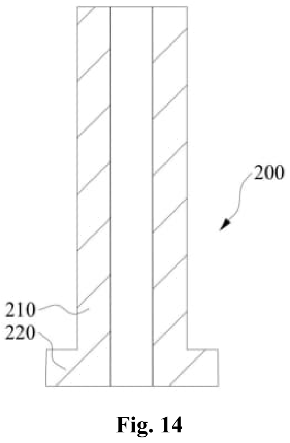

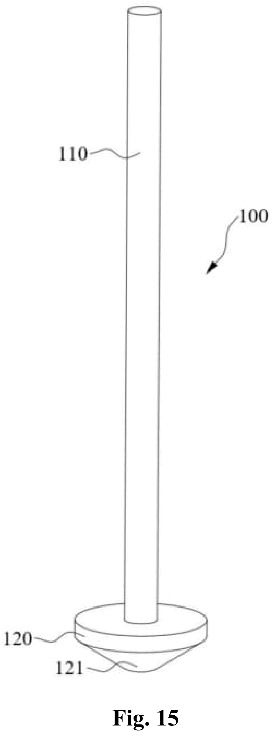

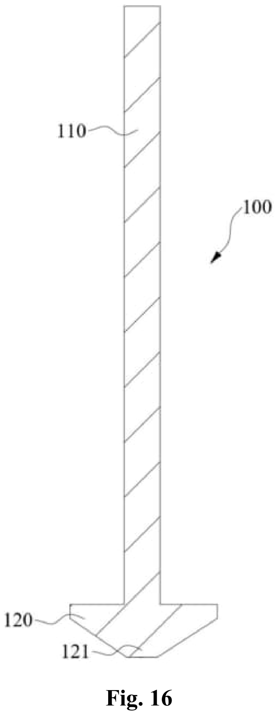

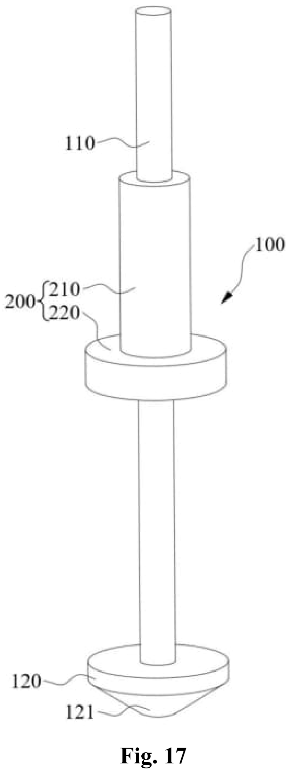

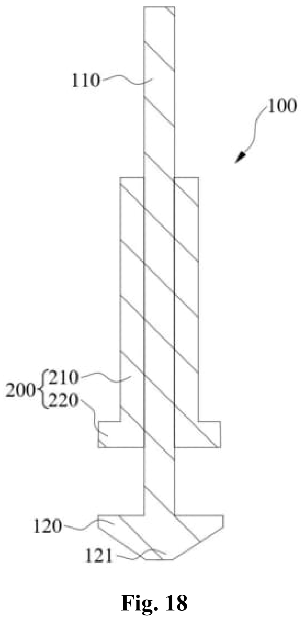

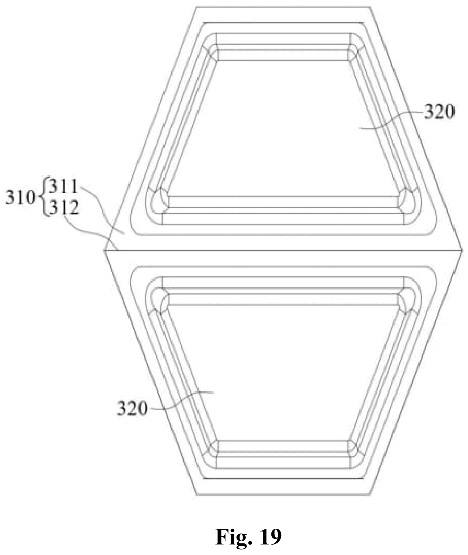

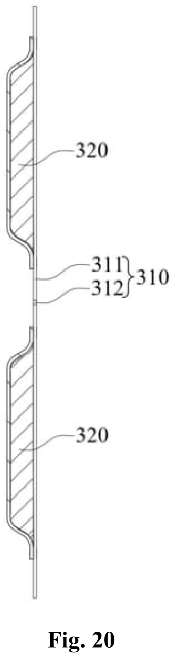

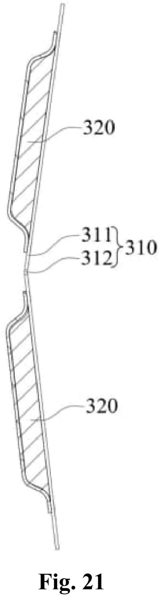

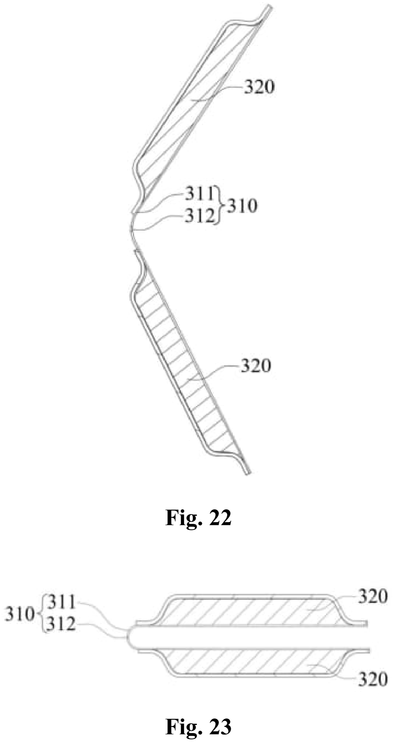

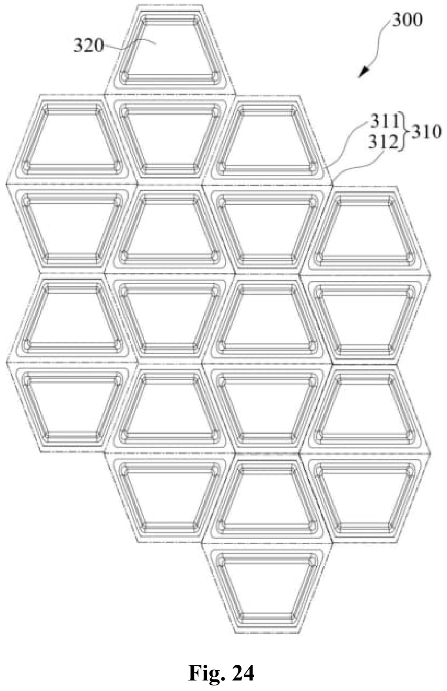

As shown in Fig. 1 to Fig. 24, this embodiment provides a compressible and foldable all-suture interface screw including the deformable fixation body 300, the pull bolt 100, and the top bolt 200, the deformable fixation body 300 includes the flexible pipe 310 and several rigid blocks 320 fixed to the outer wall of the flexible pipe 310, the flexible pipe 310 is made of a textile fabric, the rigid block320 is an isosceles trapezoidal column in shape, the thickness direction of the rigid block320 is the same as the radial direction of the flexible pipe310, the length direction of the flexible pipe 310 is perpendicular to the long side surface of the rigid pipe 310, the end surface of the rigid block 320 close to the flexible pipe 310 is a connection surface, adjacent two of the rigid blocks 320 form a folding unit, the long side surfaces of two of the rigid blocks 320 in the same folding unit are opposite to each other, and under a condition that force is applied to the folding unit along the length direction of the flexible pipe 310, the folding unit drives the flexible pipe 310 to deform and move to cause two of the connection surfaces to be close to each other; the pull bolt 100 has the bolt head 120 and the bolt rod 110 fixedly connected to one end of the bolt head 120, the bolt rod 110 passes through the flexible pipe 310 and the top bolt 200 in sequence, the top bolt 200 is slidable relative to the bolt rod 110, the length direction of the bolt rod 110 is the same as the length direction of the deformable fixation body 300, and the deformable fixation body 300 is sandwiched between the bolt head 120 and the top bolt 200.

The deformable fixation body 300 of the compressible and foldable all-suture interface screw consists of the flexible pipe 310 and the rigid blocks 320, and can be deformed and moved under a condition that force is applied along the length direction of the deformable fixation body 300, so that two connection surfaces in the folding unit are close to each other to achieve the accurate control over the deformation of the deformable fixation body 300. The rigid blocks 320 are grouped and limited by the folding unit, so that the long side surfaces of two rigid blocks 320 are provided oppositely on the hexagonal folding unit, and each folding unit is evenly distributed over the flexible pipe 310. The pull bolt 100 and the top bolt 200 serve the function of assisting the folding and deformation of the deformable fixation body 300, and by fitting to the pull bolt 100 and the top bolt 200, the deformable fixation body 300 can be sandwiched between the pull bolt 100 and the top bolt 200, which can provide desired stability and reliability for the overall structure of the compressible and foldable all-suture interface screw. The deformable fixation body 300 is fitted to the shaft holes of the pull bolt 100 and the top bolt 200, so as to assist folding and deforming of the deformable fixation body 300 based on the relative axial movement between the pull bolt 100 and the top bolt 200. After the deformation of the deformable fixation body 300 is completed, the pull bolt 100 and the top bolt 200 are withdrawn to fix the deformable fixation body 300 to the bone tunnel, and the pull bolt 100 and the top bolt 200 are withdrawn after the auxiliary operation is completed. The compressible and foldable all-suture interface screw is folded and deformed by the deformable fixation body 300, so that the diameter of the deformable fixation body 300 after deformation is increased along the radial direction, and the deformable fixation body 300 has a laminated structure along the axial direction. Further, the flexible pipe 310 is made of the textile fabric, so that the deformable fixation body 300 may be in contact with the bone tunnel through the flexible object, thereby increasing the roughness of the surface of the deformable fixation body 300, increasing the friction between the compressible and foldable all-suture interface screw and the bone tunnel, and improving the use effect. Therefore, the movement of the deformable fixation body 300 may be restricted by the friction between the bone surface and the deformable fixation body 300, so that the deformable fixation body 300 is not easily expanded after compression and folding, thereby ensuring the long-term stable operation of the deformable fixation body 300. With the design that the deformable fixation body 300 after compression and folding is in contact with the bone tunnel, the contact area between the deformable fixation body 300 and the bone tunnel is increased, the compressing force between the deformable fixation body 300 and the surface bone is increased, and the toughness of the deformable fixation body 300 itself is increased, so that the damage to the bone in practice is reduced, the self-adaptability to the bone tunnel is increased, and the fixation strength and the fixation stability are increased, thereby reducing the risk of surgical failure, helping to simplify the practical clinical surgical operation, and providing stable post-surgical repair effect. The compressible and foldable all-suture interface screw is provided with the folding unit which is fixed and stable and the compressible and foldable structure which is bi-directionally stable, ensuring the long-term stable operation of the deformable fixation body 300 with the preset deformation solution for the deformable fixation body 300.

In this embodiment, the rigid blocks 320 are distributed over the outer wall of the flexible pipe 310, and the cross section of the flexible pipe 310 is a hexagon in shape. With the arrangement of the rigid blocks 320, the stability and the durability of the entire deformable fixation body 300 are increased, and the deformation of the deformable fixation body 300 is planned, which is beneficial for ensuring that the deformable fixation body 300 deforms as expected, so as to increase the operation effect of the compressible and foldable all-suture interface screw.

Exemplarily, the flexible pipe 310 consists of the partition part 312 and the carrying part 311, the partition part 312 is for partitioning all of the carrying parts 311 one by one, each carrying part 311 is provided with one rigid block 320, the shape of each carrying part 311 is the same as the shape of the projection of the cross section of the rigid block 320 on the carrying part 311, and the dimension of each carrying part 311 is provided to scale.

The carrying parts 311 are partitioned by the partition part 312, and the rigid block 320 is provided in the carrying part 311, so that the bending position of the flexible pipe 310 is planned by providing a fold at the partition part 312. This ensures that the flexible pipe 310 may be deformed as expected, so as to ensure that the deformable fixation body 300 after deformation can form gullies and protrusions as expected. The partition part 312 serves the function of limiting the folding position of each folding unit, ensuring that the folding unit can complete bringing two connection surfaces close to each other as required. With the above design, the compressible and foldable all-suture interface screw has better adaptability and flexibility, which is beneficial for optimizing the structural layout of the deformable fixation body 300 and customizing based on actual needs.

Further, the partition part 312 and the carrying part 311 are integrally woven, one of the partition part 312 and the carrying part 311 consists of a knit stitch coil, and the other of the partition part 312 and the carrying part 311 consists of a purl stitch coil.

By differentiating the knit stitch coil from the purl stitch coil, the stability and the durability of the deformable fixation body 300 are increased, which is beneficial for ensuring the all suture design of the compressible and foldable all-suture interface screw.

Specifically, the flexible pipe 310 is a knitted weft-knitting fabric structure, and the flexible pipe 310 forms a hexagonal weave structure provided circularly with the combination of knitted weft-knitting knit stitch coil and purl stitch coil. In this embodiment, taking for the example that the partition part 312 is the knit stitch and the carrying part 311 is the purl stitch, each hexagonal weft-knitting structure forms a folding unit.

In this embodiment, the folding unit is distributed over the surface of the flexible pipe 310, so that two regions are formed: the rigid body region with the higher rigidity and the hinge region with the lower rigidity. Specifically, the hinge region is provided at the position where the partition part 312 is located, and the rigid body region is provided at the position where the carrying part 311 is located. Under a condition that the centripetal compression along the up and down directions is applied to the deformable fixation body 300 by the pull bolt 100 and the top bolt 200, the rigid body region does not deform due to the high stiffness, and the hinge region starts to yield and deform due to the low stiffness, the outer convex mountain folds outward to guide the deformation, and the inner concave valley folds inward to guide the deformation. Originally, the rigid body regions are arranged longitudinally; under this condition, the direction of the rigid body regions starts to change, and the rigid body regions are arranged laterally and stacked together, so that the deformable fixation body 300 is folded as a whole, the overall shape of the rigid body regions changes from the longitudinally elongated cylindrical shape to the laterally expanded short cylindrical shape, and the compressible and foldable all-suture interface screw is in close contact with the bone surface.

The compressible and foldable all-suture interface screw according to this embodiment uses a fully flexible fixture, and may be folded and deformed as expected. Due to the existence of the deformation solution, the shape of the deformable fixation body 300 implanted into the bone tunnel changes regularly. With this deformation solution, in one aspect, the expansion of the diameter of the deformable fixation body 300 along the radial direction of the bone tunnel is greater than the expansion of the diameter for the general flexible deformation, the contact area between the deformable fixation body 300 and the bone tunnel is increased, and the fixation strength is increased accordingly. In another aspect, compared with the flexible material that is generally disorderly deformed, the structure of the deformable fixation body 300 folded layer by layer along the axial direction of the bone tunnel can increase the toughness of the deformable fixation body 300 itself and increase the stability. In addition, the rotational implantation is not used in the compressible and foldable all-suture interface screw, so that under a condition that the compressible and foldable all-suture interface screw is subjected to the alternating load, the fixation instability caused by thread retraction happening to the screw-in implanted fixtures does not happen to the compressible and foldable all-suture interface screw. Instead, since the outer surface of the folding structure has a plurality of sets of gullies and protrusions, and the deformable fixation body 300 has a suture-like rough surface, the friction between the compressible and foldable all-suture interface screw and the bone tunnel is increased, and the adaptability to the alternating load formed by the walking is better, and the stability is higher. Therefore, the above ensures that the compressible and foldable all-suture interface screw can be a bi-directionally stable compressible and foldable structure.

In this embodiment, the outer contour of the partition part 312 enclosing one folding unit is a regular hexagon in shape. With the above design, the structural layout of the folding unit on the flexible pipe 310 is planned, the full utilization of the surface of the flexible pipe 310 is ensured, the space occupied by the flexible pipe 310 is reduced, and the force applied to the deformable fixation body 300 is optimized, which is beneficial for ensuring that the deformable fixation body 300 deforms as expected. With the above design, the structure of the folding unit is more appropriate and stable, and the reliability and the service life of the entire deformable fixation body 300 are increased.

Exemplarily, the rigid block 320 includes the filler 321 and the capsule 322, the capsule 322 is fixedly connected to the outer wall of the flexible pipe 310, the capsule 322 and the flexible pipe 310 enclose the filling cavity, and the filling cavity is filled with the filler 321. With the arrangement of the filler 321, the rigid block 320 has better supporting and fixing effects to satisfy the requirements for different interfaces. With the arrangement of the capsule 322, the risk of damage to the rigid block 320 is reduced, the risk of leakage of the filler 321 is reduced, and the service life of the rigid block 320 is increased. The filler 321 is fitted to the capsule 322, so that the space occupied by the rigid block 320 is reduced, and the production cost of the rigid block 320 is reduced, thereby ensuring the smooth forming of the rigid block 320, and ensuring the stable operation of the rigid block 320.

Each folding unit of the compressible and foldable all-suture interface screw consists of two adjacent rigid blocks 320 connected to the flexible pipe 310, and the filler 321 is made of flexible material and may be adaptively adjusted based on the actual shape of the bone tunnel, so that the connection strength and the stability of the folding unit are ensured. The above ensures that the compressible and foldable all-suture interface screw may become a structure of the fixed and stable folding unit.

Specifically, the capsule 322 is made of the knitted weft-knitting fabric, and may be directly woven into an isosceles trapezoid or may be obtained by cutting the fabric.

Further, the capsule 322 is made of the textile fabric, the rigid block 320 further includes the overlock stitch 323 for sewing the capsule 322 to the outer wall of the flexible pipe 310, and the overlock stitch 323 forms equally spaced stitching points along the edge of the capsule skin 322.

The capsule 322 is made of the textile fabric, so that the deformation capacity and the toughness of the surface of the rigid block 320 are increased, and the situation that the rigid block 320 hinders the smooth deformation is prevented; the overlock stitch 323 sews the capsule 322 to the outer wall of the flexible pipe 310, so that the rigid block 320 may be fixed to the outer wall of the flexible pipe 310 more firmly. With the above design, the stability and the durability of the structure are increased, and the fixing effect of the rigid block 320 is increased.

In this embodiment, the filler 321 is fibrous, and the volume of the filler 321 is between 30% and 80% of the maximum volume of the filling cavity.

With such arrangement, the filler 321 can fully serve the supporting and fixing function while preventing occupying excessive space, so that appropriate hardness and elasticity of the rigid block 320 can be maintained, different use requirements may be satisfied, and compactness and appropriateness of the structure of the rigid block 320 may be ensured.

Further, the filler 321 may be any fibrous material for medical use. Specifically, the filler 321 is PET flocculent fibers.

Exemplarily, the top bolt 200 is provided with the avoidance hole through the top bolt 200, and the hole wall of the avoidance hole is slidably fitted to the outer wall of the bolt rod 110.

The avoidance hole is fitted to the bolt rod 110, so that the bolt rod 110 move more smoothly in the top bolt 200, the friction resistance is reduced, the flexibility and the reliability of the entire structure are increased, which is beneficial for increasing the operation reliability of compressing the deformable fixation body 300.

In this embodiment, the top bolt 200 is the hollow tubular component, the top bolt 200 includes the bolt pipe 210 and the bolt ring 220 fixedly connected to one end of the bolt pipe 210, the cross section of the bolt ring 220 covers the cross section of the bolt pipe 210, and the deformable fixation body 300 is sandwiched between the bolt head 120 and the bolt ring 220.

The cross section of the bolt ring 220 covers the cross section of the bolt pipe 210, so that the structural optimization of the top bolt 200 is achieved, which is beneficial for reducing the production cost of the top bolt 200, reducing the occupied space, and reducing the operation difficulty of compressing the deformable fixation body 300.

Specifically, one end of the bolt head 120 away from the bolt rod 110 is provided with the frustum 121, the frustum 121 is coaxial with the bolt rod 110, and the diameter of the frustum 121 is gradually decreased along the direction away from the bolt rod 110.

In this embodiment, the deformable fixation body 300 is coaxial with the bolt rod 110, and the bolt rod 110 penetrates through the deformable fixation body 300 and the avoidance hole in sequence.

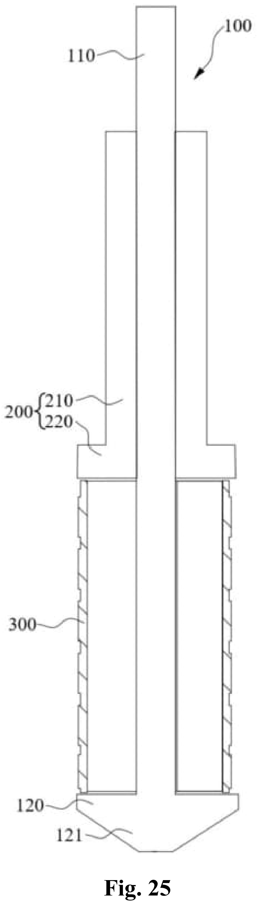

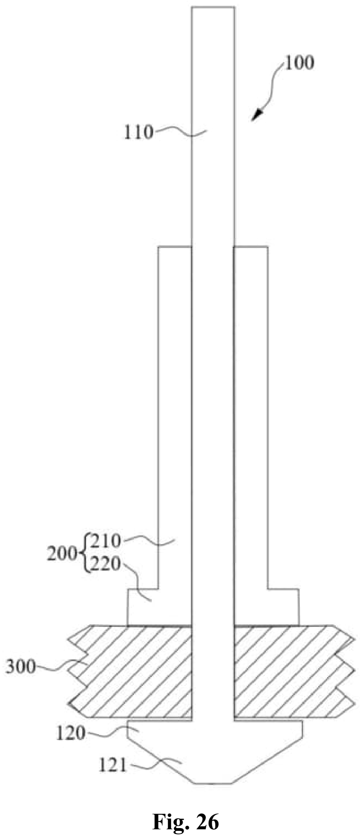

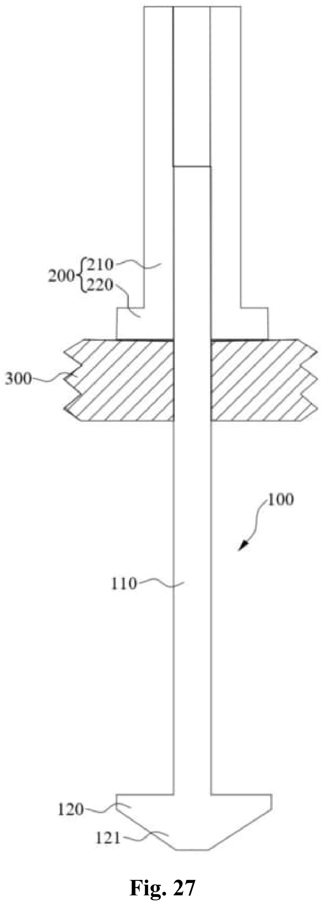

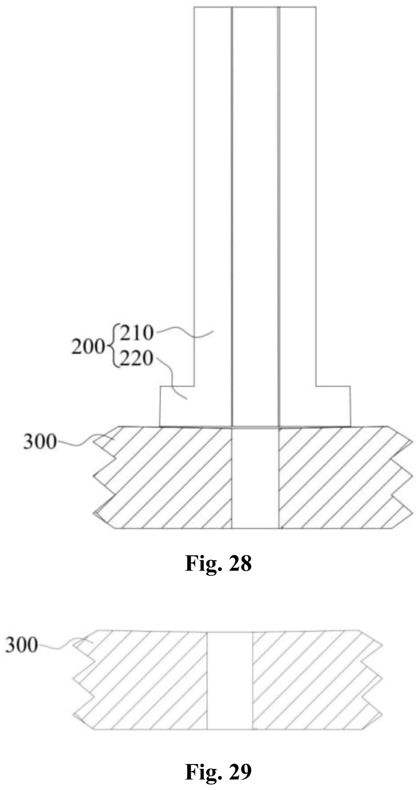

As shown in Fig. 25 to Fig. 29, this embodiment further provides a compressible and foldable all-suture interface screw implantation method, the compressible and foldable all-suture interface screw implantation method is applicable to the compressible and foldable all-suture interface screw and includes the following steps: passing through the deformable fixation body 300 and the top bolt 200 in sequence using the pull bolt 100; placing the compressible and foldable all-suture interface screw on the injured position, keeping the top bolt 200 fixed, pulling the pull bolt 100 to compress the deformable fixation body 300 and causing the deformable fixation body 300 to start to be compressed, and stopping after the pull bolt 100 moves the preset length, under this condition, completely deforming the deformable fixation body 300, where the deformation length of the deformable fixation body 300 is the preset length, and the diameter is expanded as expected; then pushing out the pull bolt 100 first, and then taking out the top bolt 200 to determine that the implantation is completed and the deformable fixation body 300 performs the function normally.

Obviously, the above embodiments of the present application are merely for the purpose of clearly illustrating the examples given in the present application, and are not intended to limit the embodiments of the present application. For those skilled in the art, based on the above description, other variations or changes in different forms may be made. All embodiments need not and cannot be exhaustive herein. Any modification, equivalent replacement, improvement, and the like made within the gist and principle of the present application shall be should be included in the protection scope of the claims of the present application.

## Claims

1. A compressible and foldable all-suture interface screw comprising:
a deformable fixation body (300), wherein the deformable fixation body (300) comprises a flexible pipe (310) and a plurality of rigid blocks (320) fixed to an outer wall of the flexible pipe (310), the flexible pipe (310) is made of a textile fabric, the rigid block (320) is an isosceles trapezoidal column in shape, a thickness direction of the rigid block (320) is same as a radial direction of the flexible pipe (310), a length direction of the flexible pipe (310) is perpendicular to a long side surface of the rigid pipe (310), an end surface of the rigid block (320) close to the flexible pipe (310) is a connection surface, adjacent two of the rigid blocks (320) form a folding unit, long side surfaces of two of the rigid blocks (320) in a same one of the folding units are opposite to each other, and under a condition that force is applied to the folding unit along the length direction of the flexible pipe (310), the folding unit drives the flexible pipe (310) to deform and move to cause two of the connection surfaces to be close to each other; and
a pull bolt (100) and a top bolt (200), wherein the pull bolt (100) has a bolt head (120) and a bolt rod (110) fixedly connected to one end of the bolt head (120), the bolt rod (110) passes through the flexible pipe (310) and the top bolt (200) in sequence, the top bolt (200) is slidable relative to the bolt rod (110), a length direction of the bolt rod (110) is same as a length direction of the deformable fixation body (300), and the deformable fixation body (300) is sandwiched between the bolt head (120) and the top bolt (200).

2. The compressible and foldable all-suture interface screw according to claim 1, wherein the rigid blocks (320) are distributed over the outer wall of the flexible pipe (310), and a cross section of the flexible pipe (310) is a hexagon in shape.

3. The compressible and foldable all-suture interface screw according to claim 1, wherein the flexible pipe (310) consists of a partition part (312) and a carrying part (311), the partition part (312) is configured to partition all of the carrying parts (311) one by one, each of the carrying parts (311) is provided with one of the rigid blocks (320), a shape of each of the carrying parts (311) is same as a shape of a projection of a cross section of the rigid block (320) on the carrying part (311), and a dimension of each of the carrying parts (311) is provided to scale.

4. The compressible and foldable all-suture interface screw according to claim 3, wherein the partition part (312) and the carrying part (311) are integrally woven, one of the partition part (312) and the carrying part (311) consists of a knit stitch coil, and the other of the partition part (312) and the carrying part (311) consists of a purl stitch coil.

5. The compressible and foldable all-suture interface screw according to claim 3, wherein an outer contour of the partition part (312) enclosing one of the folding units is a regular hexagon in shape.

6. The compressible and foldable all-suture interface screw according to claim 1, wherein the rigid block (320) comprises a filler (321) and a capsule (322), the capsule (322) is fixedly connected to the outer wall of the flexible pipe (310), the capsule (322) and the flexible pipe (310) enclose a filling cavity, and the filling cavity is filled with the filler (321).

7. The compressible and foldable all-suture interface screw according to claim 6, wherein the capsule (322) is made of a textile fabric, the rigid block (320) further comprises a overlock stitch (323) configured to sew the capsule (322) to the outer wall of the flexible pipe (310), and the overlock stitch (323) forms equally spaced stitching points along an edge of the capsule (322).

8. The compressible and foldable all-suture interface screw according to claim 6, wherein the filler (321) is fibrous, and a volume of the filler (321) is between 30% and 80% of a maximum volume of the filling cavity.

9. The compressible and foldable all-suture interface screw according to according to claim 1, wherein the top bolt (200) is provided with an avoidance hole through the top bolt (200), and a hole wall of the avoidance hole is slidably fitted to an outer wall of the bolt rod (110).

10. The compressible and foldable all-suture interface screw according to any one of claims 1 to 9, wherein the top bolt (200) is a hollow tubular component, the top bolt (200) comprises a bolt pipe (210) and a bolt ring (220) fixedly connected to one end of the bolt pipe (210), a cross section of the bolt ring (220) covers a cross section of the bolt pipe (210), and the deformable fixation body (300) is sandwiched between the bolt head (120) and the bolt ring (220).
